# EUROPÄISCHE PATENTSCHRIFT

(11) **EP 2 679 666 B1**
(45) Veröffentlichungstag und Bekanntmachung des Hinweises auf die Patenterteilung: **04.03.2015**
(21) Anmeldenummer: 13401055.2
(22) Anmeldetag: 03.06.2013
(51) Int. Cl.: C12M 3/06, C12M 1/00, C12M 1/12

(54) **Gefäßmodell, Verfahren zu seiner Herstellung und seine Verwendung**
Vascular model, method for its manufacture and application
Modèle de récipient, son procédé de fabrication et son utilisation

(30) Priorität: 26.06.2012 DE 102012105540
(43) Veröffentlichungstag der Anmeldung: 01.01.2014
(73) Patentinhaber: Karlsruher Institut für Technologie, 76131 Karlsruhe (DE)
(72) Erfinder: Giselbrecht, Stefan, Dr., 76187 Karlsruhe (DE); Hebeiß, Isabella, 74259 Widdern (DE); Schepers, Ute, Dr., 53842 Troisdorf (DE); Truckenmüller, Roman, Dr., 74223 Flein (DE)

(56) Entgegenhaltungen:
- WO-A1-2009/042671
- DE-T2- 60 112 812
- US-A1- 2011 082 563
- US-A1- 2012 318 726
- FIDDES L K ET AL: "A circular cross-section PDMS microfluidics system for replication of cardiovascular flow conditions", BIOMATERIALS, ELSEVIER SCIENCE PUBLISHERS BV., BARKING, GB, Bd. 31, Nr. 13, 18. Februar 2010 (2010-02-18), Seiten 3459-3464, XP026945980, ISSN: 0142-9612, DOI: 10.1016/J.BIOMATERIALS.2010.01.082 [gefunden am 2010-02-18]

## Beschreibung

Die Erfindung betrifft ein Gefäßmodell, ein Verfahren zu seiner Herstellung und seine Verwendung zur Kultivierung tubulärer Gewebestrukturen, insbesondere zur Nachahmung von Blut- und Lymphgefäßen, der Blut-Hirn-Schranke, von Lungen-, Magen- oder Darmepithelien und/oder von glandulären Strukturen.

Zur Simulation von Blutgefäßen und der sie umgebenden organotypischen Kultur sind bereits Modelle bekannt.

So zeigt US2011/0082563 A1 einen mikrofluidischen Bioreaktor zur Zellkultivierung vor, der aus zwei mit Kanälen versehenen Lagen besteht, die durch eine Membran voneinander getrennt sind. Auf einer Lage sind mindestens 2 Kanäle eingebracht, welche so angeordnet sind, dass sie mit dem Kanal auf der anderen Lage überlappen und über die Membran in fluidischen oder mechanischen Kontakt stehen.

Die bekannten Modelle weisen eine Vielzahl von Nachteilen auf, insbesondere im Hinblick auf die Form der Gefäße, die Dichte verschiedener Kapillaren in der organotypischen Kultur oder die Porosität von Kanälen, die die aktive Versorgung der organotypischen Kultur gewährleisten, einschließlich Gradientenbildung. Die Komplexität des vaskulären Systems erfordert jedoch eine in vivo-nahe Darstellung der physiologischen Gegebenheiten insbesondere der Krümmungsradien der Blutgefäße, Wechselwirkungen mit der Extrazellulärmatrix und Zellen oder Scherkräfte.

Mit bekannten Verfahren zur Herstellung von Mikrokanalstrukturen, insbesondere Lithographie (bevorzugt Röntgen-, UV- oder Laserstrahlung), mechanische Mikrostrukturierung, Laserstrukturierung, Soft Lithografie, Mikro-Replikationsverfahren wie Spritzguss oder Heißprägen, oder Rapid Prototyping-Verfahren werden üblicherweise mikrofluidische Kanäle mit rechteckigem Querschnitt erhalten. Zellen, die in derartigen Kanälen kultiviert werden, wachsen auf einer flachen Oberfläche, was jedoch nicht den in vivo-Gegebenheiten entspricht [1]. Mit vielen mikrotechnischen Verfahren lassen sich in aufwändiger Weise Kanäle mit rundem Querschnitt herstellen, die aber typischerweise keine Porosität aufweisen [2-6]. Poren sind jedoch erforderlich für die Versorgung von Zellen sowohl von der apikalen als auch von der basolateralen Seite und für die Untersuchung von transendothelialen Transportprozessen.

Es ist bekannt, dass ein dreidimensionales Gewebe, das in unmittelbarer Nachbarschaft eines Blutgefäßes lokalisiert ist, Faktoren sekretiert, die den transendothelialen Transport verstärken können. Das ist besonders bei Tumorgeweben relevant, da sie Proteasen sekretieren bzw. rekrutieren, die eine kurzzeitige Auflösung der endothelialen Konnektivität durch Proteolyse der sog. *Tight Junctions* fördern und somit das Blutgefäß für den Wirkstofftransport durchlässig machen.

Es konnte gezeigt werden, dass sich die Morphologie der Endothelzellen und die korrekte Ausbildung von *Tight Junctions* zwischen den Endothelzellen nicht nur unter statischen, sondern auch unter fluidischen Bedingungen in geraden rechteckigen Kanälen deutlich von der in verrundeten Kanälen unterscheidet. Weiterhin unterscheiden sich Fließprofile in rechteckigen Mikrokanälen von Fließprofilen in Mikrokanälen mit zirkulärem Querschnitt, wodurch auch die Differenzierung der Endothelzellen, die in diesen Kanälen kultiviert werden können, stark variiert [2, 7]. Auch wurden Anomalien in der Bildung und der Größe der plasmareichen Schicht durch rote Blutzellen in rechteckigen fluidischen Mikrokanälen beobachtet [8]. Morphologiestudien an Endothelzellen in Mikrokanälen mit rechteckigem und zirkulärem Querschnitt zeigten, dass es besonders starke Unterschiede in der korrekten Ausbildung des Aktinzytoskeletts und der fokalen Adhäsionspunkte gibt [9]. Dabei unterscheiden sich die Morphologie der Endothelzellen und die korrekte Ausbildung von *Tight Junctions* zwischen den Endothelzellen nicht nur unter fluidischen, sondern auch unter statischen Bedingungen deutlich.

Eine der natürlichen Umgebung entsprechende dreidimensionale Struktur repräsentiert eher die in vivo-Situation der Zellen als eine flache Oberfläche [10-12]. Da das vaskuläre System ein komplexes zelluläres System darstellt, ist es für ein in vitro-System sehr wichtig, so gut wie möglich die physiologische Umgebung, insbesondere Krümmung der Gefäßstrukturen, Zusammensetzung der extrazellulären Matrix, Fluidik, Scherkräfteverhältnis, Versorgungsdichte des dreidimensionalen Gewebes mit Kanälen in geringen Abständen, Verzweigungen der Kanäle, zu gewährleisten [12, 13]. Die meisten mikrofluidischen Kanalstruktursysteme werden durch Soft Lithography aus Polydimethylsiloxane (PDMS) hergestellt. In [2] wird ein mikrofluidisches Kanalsystem aus PDMS mit zirkulärem Querschnitt vorgestellt, mit dem sich kardiovaskuläre Fließbedingungen auf Endothelzellen imitieren und untersuchen lassen. Allerdings ist dieses System für die Untersuchungen von transendothelialem Wirkstofftransport in Gewebe nicht geeignet, da es sich um geschlossene Kanäle handelt, deren Außenhülle hauptsächlich aus einer Kollagenmatrix besteht.

Gegenwärtig existieren verschiedene artifizielle Modelle von runden perfundierten Kanälen, basierend auf Hohlfasern als artifizielle Blutgefäße [14]. Hohlfasersystemen ist jedoch gemeinsam, dass Gradienten sowohl in longitudinaler als auch radialer Richtung der Fasern auftreten können, da das Nährmedium vom Kapillareingang zum Kapillarausgang eine bestimmte Strecke zurücklegen muss, auf der die Zellen das Nährmedium an Nährstoffen verarmen. Die Bereitstellung von Ernährung und Durchblutung durch Blutgefäße ist ein wesentliches ungelöstes Problem bei der Etablierung eines organotypischen Modells. Schon bei geringen Gewebevolumina ist es wichtig, ein Gefäßsystem oder ein entsprechendes Äquivalent zu implementieren, da für Abstände von mehr als etwa 100-300 µm bis zur nächsten Blutkapillare die Diffusion zur Ernährung nicht mehr ausreicht. Es besteht daher ein Bedarf an Modellen, in denen sich versorgende Blutgefäße in Kombination mit einem beliebigen Gewebe kultivieren lassen und die zudem den Ansprüchen eines (mikro-) vaskulären Systems gerecht werden. Es ist somit die Bereitstellung einer physiologisch korrekten, dreidimensionalen Anordnung erforderlich, die mit herkömmlichen Hohlfasersystemen bisher nicht erreicht wurden, da Hohlfasern weder eine Verzweigung noch eine häufige Änderung der Querschnitte zulassen.

Systeme, wie in [19] beschrieben, beruhen auf der Neubesiedlung azellularisierter Dünndarmsegmente von Schweinen, deren vaskuläres System mit Endothelzellen neubesiedelt wird. Auf der äußeren Seite der Blutgefäße lassen sich verschiedenartige dreidimensionale Gewebe kultivieren, die über die neubesiedelten Blutgefäße versorgt werden können. Das System muss jedoch hinsichtlich der Versorgung der Gewebe weiter optimiert werden. Durch die Variabilität bei der Explantation und Azellularisierung ergeben sich unterschiedliche Qualitäten für die einzelnen Matrizen. Außerdem lässt sich dieses Modell nicht als Einmalprodukt für die Testung im Hochdurchsatzverfahren einsetzen.

In [15] wird ein sehr einfaches Konzept für den Einsatz von thermogeformten, dünnwandigen und porösen Kanälen für die Versorgung von dicken dreidimensionalen Geweben vorgeschlagen. Darin wird jedoch die aktive Durchströmung der Kanäle zur Versorgung der Zellen nicht berücksichtigt. Zellen im Hydrogel werden nur durch Diffusion aus den nicht aktiv durchströmten Kanälen versorgt.

Ausgehend hiervon ist es die Aufgabe der vorliegenden Erfindung, ein Gefäßmodell, ein Verfahren zu seiner Herstellung und seine Verwendung bereitzustellen, die die genannten Einschränkungen und Nachteile des Standes der Technik überwinden.

Diese Aufgabe wird im Hinblick auf das Gefäßmodell durch die Merkmale des Anspruchs 1, im Hinblick auf das Herstellungsverfahren durch die Schritte des Anspruchs 7 und im Hinblick auf die Verwendung des Gefäßmodells durch den Anspruch 9 gelöst. Die Unteransprüche beschreiben jeweils vorteilhafte Ausgestaltungen der Erfindung.

Erfindungsgemäß wird ein Gefäßmodell nach Anspruch 1 bereitgestellt, das mindestens einen dünnwandigen, d.h. mit einer Wanddicke von 0,1 µm bis 1000 µm, bevorzugt von 1 µm bis 100 µm, und porösen Mikrokanal mit gekrümmten Innenflächen und mindestens eine, den Mikrokanal umgebende und ebenfalls aktiv durch ein Fluid, d.h. eine Flüssigkeit oder ein Gas, worin Partikel eingebracht sein können, durchströmbare Kammer zur in vitro-Nachahmung tubulärer, insbesondere dreidimensionaler Gewebsstrukturen und dem direkt benachbarten Gewebe aufweist, d.h. insbesondere Blut- und Lymphgefäße, Blut-Hirn-Schranke, Lungen- Magen- oder Darmepithel, oder glanduläre Strukturen, z.B. Pankreas, besitzt.

Der mindestens eine Mikrokanal weist einen gekrümmten, vorzugsweise halbrunden Querschnitt auf, was die natürliche Form bzw. die natürlichen Krümmungsradien eines in vivo-Blutgefäßes widerspiegelt. Die Dimensionen des mindestens einen Mikrokanals werden entsprechend den zu modellierenden Blutgefäßen angepasst. Die fünf wichtigsten Blutgefäße sind:
- Arterien, Durchmesser größer als 1 cm bei elastischen Arterien bzw. 0,1-10 mm bei muskulösen Arterien;
- Arteriolen, Durchmesser 10 - 100 µm;
- Kapillaren, mit Durchmesser 4 - 10 µm;
- Venolen, Durchmesser 10 - 100 µm; und
- Venen, Durchmesser 0,1 mm bis zu mehr als 1 mm.

Das Gefäßmodell weist mindestens eine, bevorzugt in eine dünne Folie mit einer Wanddicke von 0,1 µm bis 1000 µm, bevorzugt von 1 µm bis 100 µm, eingebrachte gekrümmte bzw. verrundete Hohlstruktur in Form mindestens eines Mikrokanals und eine Vielzahl von Poren, die bevorzugt statistisch verteilt sind und/oder regelmäßig angeordnet sind, in der Wandung des Kanals auf. Die Poren sind vorzugsweise rund, quadratisch, sternförmig bzw. zylindrisch, einfach konisch, doppelt konisch [Sanduhrenform] oder zigarrenförmig und wird bevorzugt mittels Ionenspurtechnologie in die Folie eingebracht. Die Porengröße liegt im Bereich von 1 nm bis 100 µm, bevorzugt von 10 nm bis 10 µm, während die Porendichte im Bereich von 1 - 10⁹ Poren/cm², bevorzugt von 10⁵ - 10⁶ Poren/cm² liegt.

Die Folie für die Kanalstruktur des Gefäßmodells besteht vorzugsweise aus einem thermoplastischen Kunststoff, besonders bevorzugt aus Polymethylmethacrylat (PMMA), Polycarbonat (PC), Polyethylenterephthalat (PET), Polystyrol (PS), Polyimid (PI), Polypropylen (PP), Polyvinylidenfluorid (PVDF) oder Cycloolefincopolymer (COC oder COP). Die Folie kann bereits vor dem Formen porös ausgestaltet sein, insbesondere als Ionenspurmembran, Faservlies oder Phasenseparationsmembran.

In einer bevorzugten Ausgestaltung ist die Kanalstruktur bevorzugt durch thermisches Bonden mit einer Abdeckung (Platte), deren Dicke im Bereich der Folie liegt, verbunden, um den mindestens einen Mikrokanal dauerhaft (fest) oder lösbar zu schließen.

Der mindestens eine Mikrokanal und/oder die mindestens eine Kammer und/oder die Abdeckung ist vorzugsweise hochtransparent und besitzt eine geringe Autofluoreszenz, vorzugsweise bei den typischen Anregungswellenlängen, die für bildgebende und optische Analysesysteme verwendet werden, insbesondere für UV/VIS, IR, oder Raman.

In einer besonders bevorzugten Ausgestaltung weist die Abdeckung mindestens einen Durchbruch, vorzugsweise mehrere Durchbrüche, im Bereich des Kanallumens auf. Das Material für die Abdeckung kann aus verschiedenen Materialien gewählt werden, bevorzugt aus Kunststoff, Glas, Metall oder Keramik.

Die Ausgestaltung des mindestens einen Mikrokanals (Kanals) kann über die Länge des Kanals variieren. Hierbei sind folgende verschiedene Anordnungen bevorzugt:
- Gerade; und/oder
- verzweigt, insbesondere in Form eines Gefäßbaums; und/oder
- parallele Kanäle nebeneinander.

Vorhanden ist mindestens
- ein gerader Kanal oder mehrere parallele gerade Kanäle und/oder
- gekrümmte Kanäle mit Krümmungsradien, wobei minimale Krümmungsradien dem 0,2 fachen der lokalen Wanddicke entsprechen, so dass konkav-gekrümmte Innenflächen des Kanals Krümmungsradien in einem Bereich von 0,02 µm bis 10000 µm, bevorzugt von 5 µm bis 5000 µm, besonders bevorzugt von 10 µm bis 1000 µm aufweisen, und/oder
- verzweigte Kanäle, d.h. eine Aufteilung eines Zweiges in mindestens zwei Zweige.

Die Breite der Kanäle liegt in einem Bereich von 0,01 µm bis 10000 µm, bevorzugt von 5 µm bis 5000 µm, besonders bevorzugt von 10 µm bis 1000 µm.

Die Tiefe der Kanäle liegt in einem Bereich von 0,1 µm bis 10000 µm, bevorzugt von 5 µm bis 5000 µm, besonders bevorzugt von 10 µm bis 1000 µm.

Mindestens ein Anschluss ermöglicht die Verbindung zwischen einem Pumpsystem und dem Kanal. Der mindestens eine Anschluss ist an mindestens eine Kammer angebracht und/oder in einer besonders vorteilhaften Ausgestaltung zusätzlich auf die Abdeckung aufgebracht und mit dieser verbunden, insbesondere durch thermisches Bonden, Klebstoff, Klebeband, Ultraschallschweißen oder Laserstrahlschweißen oder wurde bereits bei der Herstellung eingebracht oder erzeugt, bevorzugt in einem Spritzgussverfahren.

Das Gefäßmodell weist mindestens eine Kammer (Kompartiment) auf, die direkt an den Kanal angrenzt. Die mindestens eine Kammer umgibt den Kanal in seiner vollen Länge und Breite oder nur Teile davon; die Höhe der mindestens einen Kammer ist begrenzt auf 1 mm, eine besonders bevorzugte Höhe beträgt von 200 µm bis 500 µm, wobei die Geometrie der mindestens einen Kammer entsprechend der Anwendung angepasst wird.

Eine bevorzugte Ausgestaltung der Kammer ist ein kreisrundes Kompartiment, das den Kanal mindestens teilweise umgibt; besonders bevorzugt ist eine Kammer, die den Kanal vollständig umgibt und eine ähnliche Geometrie wie der Kanal besitzt, jedoch sowohl die Tiefe als auch die Höhe jeweils um 500 µm, besonders bevorzugt um 300 µm größer ist.

In einer besonderen Ausgestaltung umgibt mindestens eine zweite Kammer entsprechend den Vorgaben der ersten Kammer diese mindestens teilweise, so dass eine doppelt oder mehrfach ineinander geschachtelte Struktur entsteht.

In einer alternativen Ausgestaltung ist eine Kammer in mehrere, kleinere Kompartimente unterteilt, die jeweils nur Teilbereiche der Kanalstruktur umgeben.

Ein erfindungsgemäßes Gefäßmodell weist weiterhin mindestens einen Anschluss zur Aufnahme und/oder Abgabe des mindestens einen Fluids auf.

In einer besonders bevorzugten Ausgestaltung besitzt sowohl die Abdeckung, mit der der mindestens eine Kanal in einer bevorzugten Ausgestaltung verschlossen ist, als auch der mindestens eine Mikrokanal mindestens einen Anschluss, der insbesondere in Form eines Durchbruchs ausgestaltet sein kann und der einen Zugang von außen bzw. eine Verbindung des Kompartiments mit einem weiteren Kreislaufsystem mit mindestens einer Pumpe ermöglicht. Bei mindestens zwei Anschlüssen ist eine kontinuierliche Durchströmung der mindestens einen Kammer möglich. Ist mehr als ein Anschluss vorhanden, so sind die Anschlüsse in vergleichbarer Weise, bevorzugt über Durchbrüche, mit weiteren Zugängen versehen und vorzugsweise axial zentriert fluchtend übereinander positioniert.

Ein erfindungsgemäßes Verfahren zur Herstellung eines erfindungsgemäßen Gefäßmodells umfasst mindestens die folgenden Schritte:
- Herstellen mindestens eines Mikrokanals mit teilweise kreisförmigem Querschnitt durch Umformen einer Folie in eine Hohlstruktur, die eine Wanddicke von 0,1 µm bis 1000 µm besitzt, wobei die Folie vor dem Umformen bereits eine Vielzahl von Poren aufweist und/oder nach dem Umformen eine Vielzahl von Poren in die Folie eingebracht oder in der Folie erzeugt werden, und
- Anbringen mindestens einer Kammer an den mindestens einen Mikrokanal derart, dass diese den mindestens einen Mikrokanal in seiner vollen Länge und Breite oder in Teilen davon umgibt.

In einem bevorzugten Verfahren wird hierzu Mikrothermoformen zum Formen der Kanalstruktur eingesetzt. Das Umformen der Folie erfolgt hierbei im entropieelastischen Zustand und nicht in einer Schmelzphase, so dass ggf. eine Konservierung von Vorbehandlungen, insbesondere einer Schwerionenbestrahlung, ermöglicht wird. Die Schwerionenbestrahlung gewährleistet ein Durchdringen der Folie. Der Bestrahlungswinkel wird bevorzugt senkrecht zur Oberfläche gewählt, Masken dienen zur lokalen Begrenzung des zu bestrahlenden Bereichs oder zur lokal begrenzten Öffnung der Poren beim Ätzschritt.

Auf diese Weise lässt sich eine Kanalstruktur mit teilweise kreisförmigem, bevorzugt halbkreisförmigem Querschnitt herstellen. Im Falle der Schwerionenbestrahlung erfolgt anschließend ein chemisches Ätzen zur Auflösung der physikalisch veränderten Bereiche innerhalb der Folie, der sogenannten latenten Ionenspuren. Die Anpassung der Porengröße an das gewählte Experiment, z.B. für den Durchtritt von Molekülen oder Zellen, erfolgt hierbei durch Variation der Ätzparameter.

In einer alternativen Ausgestaltung werden bereits poröse Folien oder schichtartige Strukturen, insbesondere Vliese oder Phasenseparationsmembranen eingesetzt, die zu den gewünschten Kanalstrukturen geformt werden.

In einem besonders bevorzugten Verfahren erfolgt ein thermisches Bonden einer zweiten, porösen oder vorzugsweise nicht-porösen Polymerfolie zum Abdecken des Kanals, die in einer bevorzugten Ausgestaltung mit den entsprechenden Löchern für die fluidische Kontaktierung versehen ist. Alternativ werden hierbei andere Verbindungsverfahren, insbesondere Ultraschallschweißen, Laserschweißen oder Kleben, eingesetzt.

Schließlich wird der mindestens eine Mikrokanal bevorzugt an mindestens ein externes Pumpsystem zur Versorgung der Zellen mit Medium, zur Untersuchung von Reaktionen der Zellen auf angelegte Scherkräfte, angeschlossen. Auf diese Weise lässt sich die in vivo-Situation im Gefäßmodell imitieren.

Bevorzugt erfolgt die Herstellung einer Kammer aus mehreren Modulen, insbesondere aus einem ringförmigen Abstandshalter aus biokompatiblem Material, bevorzugt Kunststoff, Glas, Silizium oder Keramik, und einer vorzugsweise kreisförmigen, dünnen, transparenten Glasscheibe oder Kunststofffolie, die insbesondere eine geringe Eigenfluoreszenz bei den typischen Anregungswellenlängen für bildgebende und optische Analysesysteme (UV/Vis, IR, Raman usw.) aufweist. Die Verbindung des Abstandshalters mit der Folie, in die der mindestens eine Kanal eingebracht ist, und dem Material, aus dem die Kammer gebildet ist, erfolgt durch gängige Verfahren, insbesondere Kleben, thermisches Bonden, Ultraschall- oder Laserschweißen.

Ein besonders bevorzugtes Herstellungsverfahren ist das Thermoformen; hierbei lassen sich transparente, dünne Ein- oder Mehrschichtfolien in die entsprechende Form bringen. Durch Wahl geeigneter Polymere und Schichten, auch anorganische oder metallische Schichten, werden Gaspermeabilität, insbesondere für Sauerstoff, und Wasserdampfdurchlässigkeit sowie weitere Eigenschaften, wie optische Transparenz oder Elastizität, kontrolliert.

In einer besonders bevorzugten Ausgestaltung sind sowohl die Glasscheibe als auch Kunststofffolie der mindestens einen Kammer ebenfalls mit weiteren Durchbrüchen für Anschlüsse sowie mit Poren, die statistisch verteilt oder regelmäßig und/oder definiert angeordnet sind, versehen, um einen Austausch von Gasen, Flüssigkeiten, Partikeln, Zellen usw. mit der Umgebung oder mit die betreffende Kammer umhüllenden weiteren Kammern auch auf der zur Kanalfolie abgewandten Seite des Gefäßmodells zu ermöglichen.

Die erfindungsgemäße Vorrichtung weist insbesondere die folgenden Vorteile auf. Auch eine serielle, mehrfache Besiedlung, evtl. auch mit verschiedenen Zelltypen, wird möglich, ebenso eine aktive Versorgung der dreidimensional kultivierten Zellen in der umgebenden Kammer durch aktive Durchströmung dieser Kammer.

Der Stoffaustausch zwischen der umgebenden Kammer und dem Lumen der Mikrokanäle erfolgt durch die porösen Wandungen der Kanäle in beide Richtungen zur Untersuchung von Stofftransport, Transport von Signal- und Botenstoffen, Wirkstoffen, Viren und Bakterien, partikulären und faserförmigen Fremdkörpern, Migration von Zellen usw. Abhängig von der Porengröße ist hierbei eine direkte Interaktion und ein physischer Kontakt zwischen den Zellen im Lumen und den Zellen in der Kammer oder nur der Austausch von löslichen Faktoren möglich. Über die poröse, dünne Wandung des Kanals und einer sich ggf. darauf befindlichen Zellschicht lässt sich ein physikalischer und/oder chemischer Gradient zwischen dem Kanallumen und dem umgebendem Kompartiment erzeugen.

Eine aktive Durchströmung der Umgebung des mindestens einen porösen Mikrokanals durch die Erzeugung einer perfundierbaren Kammer wird möglich. Ebenso lässt sich die umgebende Kammer zur Kultivierung von künstlichen, dreidimensionalen Geweben nutzen, insbesondere mittels eingebrachten Hydrogelen, (elektrogesponnen) Fasern, Vliesen, textilen Strukturen, oder porösen schwammartigen Strukturen zur Nachahmung einer biologischen Einheit, bestehend aus tubulärer oder gefäßartiger Struktur und umgebenden Gewebe.

Ein unteres Kompartiment ermöglicht die Versorgung von Endothelzellen im porösen Mikrokanal zusätzlich von der basolateralen Seite. Dies schafft die Möglichkeit zur Ko-Kultivierung verschiedener Zelltypen, der Darstellung unterschiedlicher Gewebearten, die in Verbindung mit dem Blutgefäßsystem stehen, z.B. Bindegewebszellen. Eine Kultivierung von Tumorzellen im unteren Kompartiment zur Darstellung von Tumorgewebe in Form eines Krebsmodells wird möglich. Das untere Kompartiment kann sowohl statisch als auch aktiv betrieben werden, z.B. durch Abführen einer Art Lymphe. Möglich ist auch ein direkter Anschluss einer Analytik-Anlage (Massenspektrometrie, Raman, IR) zur Detektion des transendothelialen Transports oder von Metabolismusprozessen innerhalb des vaskulären Systems.

Das erfindungsgemäße Modell erlaubt eine quasi in vivo-Darstellung eines Blutgefäßsystems *in vitro.* Dabei ermöglicht eine Variation von Größe und Form der Kanalstrukturen eine Darstellung unterschiedlicher Gefäßarten (Arterien, Venen, Arteriolen, Kapillaren). Ein mikrofluidischer Einsatz erlaubt ein Imitieren des Blutflusses innerhalb des Blutgefäßes/des Gewebes. Damit wird eine Untersuchung des Endothels unter fluidischen Bedingungen sowie transendothelialer Prozesse ermöglicht.

Durch den Einsatz von transparenten Materialien, durch eine dünnwandige Konstruktion und durch die äußeren Abmessungen wird ein kurzzeitiges oder längerfristiges Betreiben und Mikroskopieren des Modells auf einem Standardmikroskoptisch mit Inkubationssystem ermöglicht. Die Transparenz der Bauteile erlaubt eine direkte Visualisierung transendothelialer Transportprozesse mittels Mikroskopie.

Besonders vorteilhaft ist der einfache und reproduzierbare Aufbau des erfindungsgemäßen Gefäßmodells, der eine kostengünstige Herstellung großer Stückzahlen ermöglicht und damit das Modell als kostengünstiges Einwegsystem insbesondere für Screenings zugänglich macht. Unabhängig davon lässt sich das Modell aufgrund aktiver und dynamischer Durchströmung der Kanäle und/oder Kammern mit entsprechender Analytik, insbesondere mit vor- und/oder nachgeschalteten Durchflusszellen für Infrarot, Raman usw., kombinieren.

Bevorzugte Verwendungen liegen in der Untersuchung von Wirkstoffen, Halbwertszeiten oder Blutzirkulationszeiten und der endotheliale Aufnahme oder des transendothelialen Transports verschiedener Substanzen.

Mit dem vorliegenden Gefäßmodell lassen sich tubuläre Strukturen des Körpers imitieren, insbesondere glanduläre Strukturen, Darmepithel, Lungenepithel oder Nierenkanäle, aber auch ein Modell der Blut-Hirnschranke ist denkbar. Das Modell ermöglicht die Untersuchung von Entzündungsreaktionen, z.B. die Chemokin-/Zytokin- abhängige transendotheliale Migration von Blutzellen (Leukozyten, Monozyten usw.)

Die Erfindung wird im Folgenden anhand eines Ausführungsbeispiels und der **Figur** näher erläutert.

Die **Figur** zeigt eine schematische, nicht maßstäbliche Darstellung eines erfindungsgemäßen Gefäßmodells mit einem artifiziellen Blutgefäß, das in Form eines Mikrokanals **10** ausgestaltet ist, der von einer angrenzenden Kammer (Kompartiment) **20** umgeben ist, wobei der Mikrokanal **10** und die Kammer **20** von voneinander unabhängigen Kreisläufen durchströmt werden.

Der Mikrokanal **10** weist eine Wandung in Form einer Hohlstruktur **11** auf, in die eine Vielzahl von Poren **12** eingebracht ist. Der Mikrokanal **10** wird von einem Fluid in Richtung **15** → **16** durchströmt, wodurch sich ein erster Kreislauf ausbildet.

Die Kammer **20,** die den Mikrokanal **10** umgibt, weist eine transparente Bodenplatte **21** sowie zwei Anschlüsse **31, 32** auf, wodurch sich ein zweite Kreislauf ausbildet, der ein Fluid in Richtung **31** → **32** bewegt.

Ein mikrofluidisches Perfusionssystem wird mit dem Mikrokanal **10** und der Kammer **20** derart verbunden, um die Versorgung der im Kanal enthaltenen Zellen **1-4** bzw. zur Ableitung von Lymphe oder der transendothelial transportierten Analyten zur Detektion des transendothelialen Transports zu ermöglichen.

Der Mikrokanal **10** ist mit einer transparenten Abdeckung **40** versehen, die zum einen den Mikrokanal **10** lösbar verschließt und zum anderen die Kammer **20** teilweise begrenzt.

Der vorliegende Aufbau des Gefäßmodells und die getroffene Wahl von transparenten Materialien für die einzelnen Komponenten ermöglicht die Beobachtung von Vorgängen innerhalb des Gefäßmodells mittels der Objektive **50, 50'.**

In der **Figur** ist beispielhaft eine erfindungsgemäße Verwendung des Gefäßmodells aufgezeigt. Hierbei wachsen Endothelzellen **1,** organ-spezifische Zellen **2,** Fibroblasten **3** und/oder eine biologisch abbaubare Matrix **4** an unterschiedlichen Stellen im Gefäßmodell und bilden ein artifizielles, versorgendes Blutgefäßsystem aus, das die Verteilung des Perfusionsmediums und somit die Versorgung der den Mikrokanal **10** umgebenden organotypischen 3D-Zellkultur in der Kammer **20** übernimmt.

Die Besiedlung erfolgte durch Beimpfung mit suspendierten Zellen, ggf. mehrfach, ggf. sequentiell, wobei zuerst Endothelzellen für die Gefäße, dann mesenchymale Zellen oder ähnliche organotypische Zellkulturen in die Kammer **20** eingebracht wurden. Diese Art der Besiedlung lässt sich auf nahezu beliebige vaskularisierte organotypische Zellkulturen anwenden.

Das beispielhafte Gefäßmodell weist eine mittels der sog. SMART Technologie [20] thermogeformte Polycarbonat-Folie (PC-Folie) mit einer Dicke von 65 µm auf. Das Thermoformen erfolgte bei einer Formtemperatur von 157°C und einem Gasdruck von 2,6 MPa; die Entformtemperatur betrug 70°C.

Nach dem Thermoformen wies die Membran an der dünnsten Stelle eine Dicke von 25 µm auf. Der Mikrokanal **10** wies folgende Dimensionen auf:
- Länge 20 mm,
- Breite 1 mm,
- Tiefe 250 µm.

Es wurden gerade oder verzweigt Kanäle hergestellt; Breite und Tiefe wurden variiert.

Die thermogeformte Folie wurde mit Natronlauge chemisch geätzt, um die Poren zu öffnen. Das Ätzen erfolgte über 4,5 h. Es wurden Porengrößen von 4-8 µm bei einer Porendichte von 10⁶ Poren/cm² erhalten.

Das Bonden der porösen thermogeformten PC-Folie erfolgte mit einer nicht-porösen PC-Folie, um den Kanal mittels einer Abdeckung **40** zu schließen, bei einer Temperatur von 147°C. Die gebondete thermogeformte Folie wurde an einen Block aus PMMA mit zuvor eingefrästen Luer-Anschlüssen geklebt, die den Anschluss eines Schlauchsystems ermöglichen. Über die Luer-Anschlüsse ließen sich Mikroanal **10** und/ oder die Kammer **20** befüllen. Eine Kammer **20** mit einer Höhe von 500 µm und einem Durchmesser von 24 mm wurde durch Ankleben einer Silikonringes und Verschließen mit einem Deckgläschen angefügt.

Zur Fibronektin-Beschichtung der Kanalinnenfläche wurde eine Fibronektin-Lösung mit einer Konzentration von 1 mg/ml in dest. Wasser in den Kanal gegeben und für 4 h bei Raumtemperatur inkubiert. Das Waschen des Kanals erfolgt mit PBS (Phosphate Buffered Saline). Der Kanal und die untere Kammer wurden mit Medium befüllt und über Nacht im Brutschrank bei 37°C und 5% CO₂ konditioniert.

Eine Zellsuspension humaner Nabelschnur-Endothelzellen (*Human Umbilical Vein Endothelial Cells,* HUVECs) **1** mit 5 x 10⁶ Zellen/ml wurde direkt über die Anschlüsse in den Kanal gegeben und für 10 min im Brutschrank inkubiert. Dann wurde erneut eine Zellsuspension in den Kanal gegeben, das gesamte Gefäßmodell um 90° gedreht und wieder für 10 min inkubiert. Dies erfolgte noch zwei weitere Male, um eine vollständige Besiedlung des Mikrokanals **10** mit Endothelzellen **1** zu gewährleisten. Nach dem Einbringen der Endothelzellen **1** in den Mikrokanal **10** wurden diese für weitere 3 h kultiviert, um deren festes Anwachsen auf der Hohlstruktur **11** zu ermöglichen. Anschließend wurde der Kanal an ein Pumpensystem mit Mediumreservoir angeschlossen, um die Versorgung der Zellen mit frischem Medium zu gewährleisten. Die Kammer **20** wurde entweder statisch betrieben oder mittels der Anschlüsse **31, 32** an ein Pumpsystem angeschlossen. Die Kultivierung der Endothelzellen **1** im Mikrokanal **10** erfolgte unter fluidischen Bedingungen (500 µl/min). Hierdurch erzeugte Scherkräfte entsprachen den vorhandenen Scherkräften in Blutgefäßen *in vivo.*

Als Ergebnis ergab sich zum einen nach drei- oder mehrtägiger Kultivierung unter fluidischen Bedingungen eine Ausrichtung der Endothelzellen **1** in Richtung des Medienflusses **15** → **16** im Mikrokanal **10**. Dieses Ergebnis entspricht der *in vivo*-Situation [16, 17]. Es zeigte sich zum anderen eine Ausbildung von Stress-Fibern in Flussrichtung **15** → **16** als Reaktion auf die auftretenden Scherkräfte. Unter *Stress-Fibern* werden dicke Bündel von Aktin-Filamenten, vernetzenden Proteinen und Myosin II verstanden [18].

### Literaturverzeichnis

[1] S. Srigunapalan, C. Lam, A. R. Wheeler, C. A. Simmons, Biomicrofluidics 2011, 5, 13409.
[2] L. K. Fiddes, N. Raz, S. Srigunapalan, E. Tumarkan, C. A. Simmons, A. R. Wheeler, E. Kumacheva, Biomaterials 2010, 31, 3459.
[3] J. Shao, L. Wu, J. Wu, Y. Zheng, H. Zhao, Q. Jin, J. Zhao, Lab Chip 2009, 9, 3118.
[4] J. T. Borenstein, H. Terai, K. R. King, E. J. Weinberg, M. R. Kaazempur-Mofrad, J. P. Vacanti, Biomedical Microdevices 2002, 4, 167.
[5] M. B. Esch, D. J. Post, M. L. Shuler, T. Stokol, Tissue Eng Part A 2011.
[6] K. R. King, C. C. J. Wang, M. R. Kaazempur-Mofrad, J. P. Vacanti, J. T. Borenstein, Advanced Materials 2004, 16, 2007.
[7] R. Lima, S. Wada, S. Tanaka, M. Takeda, K. Tsubota, T. Ishikawa, T. Yamaguchi, World congress on medical physics and biomedical engineering. Berlin Heidelberg: Springer 2006, 283.
[8] R. Lima, S. Wada, S. Tanaka, M. Takeda, T. Ishikawa, K. Tsubota, Y. Imai, T. Yamaguchi, Biomed Microdevices 2008, 10, 153.
[9] M. D. Frame, I. H. Sarelius, Microcirculation 2000, 7, 419.
[10] A. Abbott, Nature 2003, 424, 870.
[11] J. Deutsch, D. Motlagh, B. Russell, T. A. Desai, J Biomed Mater Res 2000, 53, 267.
[12] N. Sadr, M. Zhu, T. Osaki, T. Kakegawa, Y. Yang, M. Moretti, J. Fukuda, A. Khademhosseini, Biomaterials 2011, 32, 7479.
[13] A. D. van der Meer, A. A. Poot, M. H. Duits, J. Feijen, I. Vermes, J Biomed Biotechnol 2009, 2009, 823148.
[14] R. A. Knazek, P. M. Gullino, P. O. Kohler, R. L. Dedrick, Science 1972, 178, 65.
[15] M. Ikeuchi, K. Ikuta, in Transducers & Eurosensors '07, Lyon, France, 2007, 1337.
[16] B. Langille, S. Adamson, Circulation Research 1981, 48, 481.
[17] R. M. Nerem, M. J. Levesque, J. F. Cornhill, J Biomech Eng 1981, 103, 172.
[18] K. Katoh, Y. Kano, S. Ookawara, Vasc Health Risk Manag 2008, 4, 1273.
[19] J. Schanz, J. Pusch, J. Hansmann, H. Walles, Vascularised human tissue models: a new approach for the refinement of biomedical research, J Biotechn 2010, 148, 56.
[20] Giselbrecht, S., Gietzelt, T., Gottwald, E., Trautmann, C., Truckenmüller, R., Weibezahn, K.F., und Welle, A., 3D tissue culture substrates produced by microthermoforming of pre-processed polymer films, Biomed Microdevices 2006, 8, 191-199.

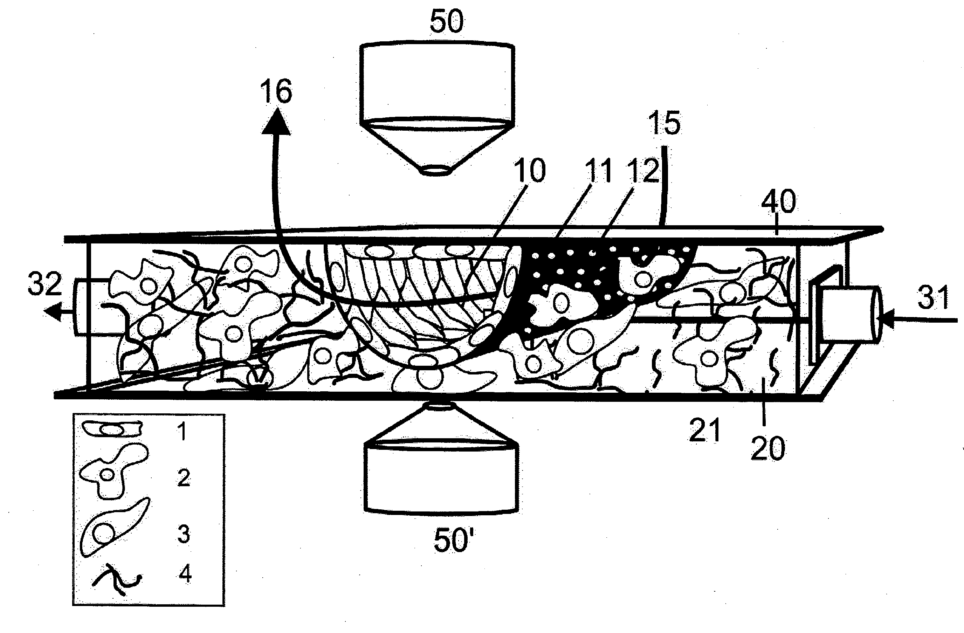

## Patentansprüche

1. Gefäßmodell, umfassend
- mindestens einen, durch mindestens ein Fluid durchströmbaren Mikrokanal (10), wobei
- der Mikrokanal in Form einer Hohlstruktur (11) mit einer Wanddicke von 0,1 µm bis 1000 µm ausgestaltet ist, die eine konkav gekrümmte Innenseite mit einem teilweise kreisförmigen Querschnitt aufweist,
- die Breite des mindestens einen Mikrokanals (10) in einem Bereich von 0,01 µm bis 10 mm liegt,
- die Tiefe des mindestens einen Mikrokanals (10) einen Wert von 0,01 µm bis 10 mm annimmt, und
- in die Hohlstruktur eine Vielzahl von Poren (12) in der Wandung des Kanals eingebracht sind, deren Durchmesser von 1 nm bis 100 µm beträgt,
- mindestens eine, durch dasselbe und/oder ein anderes Fluid durchströmbare Kammer (20), die den mindestens einen Mikrokanal (10) in seiner vollen Länge und Breite oder in Teilen davon umgibt, wobei mindestens eine Kammer (20) an den mindestens einen Mikrokanal (10) angrenzt, und
- mindestens einen Anschluss (31, 32) zur Aufnahme und/oder Abgabe des mindestens einen Fluids.

2. Gefäßmodell nach Anspruch 1, wobei der mindestens eine Mikrokanal (10) gerade ist und/oder einen Krümmungsradius im Bereich von 0,01 µm bis 10000 µm besitzt und/oder mindestens eine Verzweigung in mindestens zwei Zweige aufweist.

3. Gefäßmodell nach Anspruch 1 oder 2, wobei die Hohlstruktur (11) aus einer Folie aus einem thermoplastischen Kunststoff gebildet wird.

4. Gefäßmodell nach einem der Ansprüche 1 bis 3, wobei die Hohlstruktur (11) aus einer Ionenspurmembran, einem Faservlies oder einer Phasenseparationsmembran gebildet wird.

5. Gefäßmodell nach einem der Ansprüche 1 bis 4 mit mindestens zwei Kammern (20), wobei
- mindestens eine zweite Kammer jeweils eine erste Kammer, die den mindestens einen Mikrokanal (10) derart zumindest teilweise umgibt, dass eine ineinander geschachtelte Struktur ausgebildet ist, und/oder
- jede der mindestens zwei Kammern jeweils nur Teilbereiche des mindestens einen Mikrokanals (10) umgeben.

6. Gefäßmodell nach einem der Ansprüche 1 bis 5, wobei der mindestens eine Mikrokanal (10) mit einer Abdeckung (40) versehen ist, die den mindestens einen Mikrokanal (10) fest oder lösbar verschließt.

7. Verfahren zur Herstellung eines Gefäßmodells nach einem der Ansprüche 1 bis 6, mit den Schritten:
- Herstellen mindestens eines Mikrokanals (10) mit teilweise kreisförmigem Querschnitt durch Umformen einer Folie in eine Hohlstruktur (11) mit einer Wanddicke von 0,1 µm bis 1000 µm, wobei die Folie vor dem Umformen bereits mindestens eine Pore (12) aufweist und/oder nach dem Umformen mindestens eine Pore (12) in die Folie eingebracht oder in der Folie erzeugt wird, und in die Hohlstruktur eine Vielzahl von Poren (12) in die Wandung des Kanals eingebracht sind, deren Durchmesser von 1nm bis 100µm beträgt.
- Bereitstellen mindestens einer Kammer (20), und
- Einbringen des mindestens einen Mikrokanal in seiner vollen Länge und Breite oder in Teilen (10) in die Kammer (20).

8. Verfahren nach Anspruch 7 mit dem weiteren Schritt:
- Aufbringen einer Abdeckung (40) auf den mindestens einen Mikrokanal (10) zum dauerhaften oder lösbaren Schließen des mindestens eines Mikrokanals (10).

9. Verwendung eines Gefäßmodells nach einem der Ansprüche 1 bis 6 zur Kultivierung tubulärer Gewebestrukturen auf der konkav gekrümmten Innenseite des mindestens einen Mikrokanals (10) und/oder auf der konvex gekrümmten Außenseite des mindestens einen Mikrokanals (10) und/oder in der mindestens einen Kammer (20).

10. Verwendung nach Anspruch 9 zur Nachahmung von Blut- und Lymphgefäßen, der Blut-Hirn-Schranke, von Lungen-, Magen- oder Darmepithelien und/oder von glandulären Strukturen.

## Claims

1. Vascular model, comprising
- at least one microchannel (10), flowed through by at least one fluid, wherein the microchannel is arranged in the form of a hollow structure (11) with a wall thickness of 0.1 µm to 1000 µm, which exhibits a concave curved inner side with a partially circular cross-section,
- the width of the at least one microchannel (10) lies in a range from 0.01 µm to 10 mm,
- the depth of the at least one microchannel (10) assumes a value of 0.01 µm to 10 mm, and
- a plurality of pores (12) are introduced into the hollow structure in the wall of the channel, of which the diameter ranges from 1 nm to 100 µm,
- at least one chamber (20) which can be flowed through by the same and/or another fluid, which surrounds the at least one microchannel (10) in its full length and width or parts thereof, whereby at least one chamber (20) delimits the at least one microchannel (10), and
- at least one connection (31, 32) for taking up and/or emitting the at least one fluid.

2. Vascular model according to claim 1, wherein the at least one microchannel (10) is straight and/or exhibits a curvature radius in the range from 0.01 to 10000 µm and/or at least one branching into at least two branches.

3. Vascular model according to claim 1 or 2, wherein the hollow structure (11) is formed from a film from a thermoplastic plastic material.

4. Vascular model according to any one of claims 1 to 3, wherein the hollow structure (11) is formed from an ion track membrane, a non-woven fabric, or a phase separation membrane.

5. Vascular model according to any one of claims 1 to 4, with at least two chambers (20), wherein
- at least one second chamber in each case at least partially surrounds a first chamber, which surrounds the at least one microchannel (10), in such a way that a nested structure is formed, and/or
- each of the at least two chambers in each case surrounds only part regions of the at least one microchannel (10).

6. Vascular model according to any one of claims 1 to 5, wherein the at least one microchannel (10) is provided with a cover (40), which closes the at least one microchannel (10) securely or in a releasable manner.

7. Method for the manufacture of a vascular model according to any one of claims 1 to 6, with the steps:
- production of at least one microchannel (10) with partially circular cross-section, by forming of a film into a hollow structure (11) with a wall thickness of 0.1 µm to 1000 µm, wherein the film already comprises, before the forming, at least one pore (12) and/or, after the forming, at least one pore (12) is introduced into the film or is produced in the film, and
- in the hollow structure, a plurality of pores (12) are introduced into the wall of the channel, the diameter of which ranges from 1 nm to 100 µm,
- the provision of at least one chamber (20), and
- the introduction of the at least one microchannel in its full length and width or in parts (10) into the chamber (20),

8. Method according to claim 7 with the further step:
introduction of a cover (40) onto the at least one microchannel (10) for the permanent or releasable closure of the at least one microchannel (10).

9. Application of a vascular model in accordance with any one of claims 1 to 6, for the cultivation of tubular tissue structures on the concave curved inner side of the at least one microchannel (10) and/or on the convex curved outer side of the at least one microchannel (10) and/or in the at least one chamber (20).

10. Application according to claim 9 for the imitation of blood and lymph vessels, the blood-brain barrier, lung, stomach, or intestinal epithelia, and/or of glandular structures.

## Revendications

1. Modèle vasculaire comprenant :
- au moins un micro-canal (10) pouvant être parcouru par au moins un fluide, dans lequel :
- le micro-canal est conformé sous la forme d'une structure creuse (11) ayant une épaisseur de paroi de 0,1 µm à 1000 µm et qui comporte une face interne à courbure concave ayant une section partiellement circulaire,
- la largeur du micro-canal (10) est située dans une plage de 0,01 µm à 10 mm,
- la profondeur du micro-canal (10) a une valeur de 0,01 µm à 10 mm, et
- dans la structure creuse sont réalisées plusieurs pores (12), dans la paroi du canal, dont le diamètre est situé dans la plage de 1 nm à 100 µm,
- au moins une chambre (20) pouvant être parcourue par le même et/ou par un autre fluide et qui entoure le micro-canal (10) sur la totalité de sa longueur et de sa largeur ou sur des parties de celles-ci, au moins une chambre (20) étant adjacente au micro-canal. (10), et
- au moins une connexion (31, 32) pour l'alimentation et/ou l'extraction du fluide.

2. Modèle vasculaire conforme à la revendication 1,
dans lequel
le micro-canal (10) est rectiligne et/ou présente un rayon de courbure situé dans la plage de 0,01 µm à 10000 µm et/ou comprend au moins un embranchement en au moins deux branches.

3. Modèle vasculaire conforme à la revendication 1 ou 2,
dans lequel
la structure creuse (11) est réalisée dans une feuille en un matériau synthétique thermoplastique.

4. Modèle vasculaire conforme à l'une des revendications 1 à 3,
dans lequel
la structure creuse (11) est formée par une membrane de traçage des ions une nappe de fibres non tissées ou une membrane de séparation de phases.

5. Modèle vasculaire conforme à l'une des revendications 1 à 4,
comprenant au moins deux chambres (20) dans laquelle
au moins une seconde chambre entoure respectivement au moins partiellement une première chambre qui entoure le micro-canal (10) de façon à former une structure emboîtée, et/ou
- chacune des deux chambres n'entoure respectivement que des zones partielles du micro-canal (10).

6. Modèle vasculaire conforme à l'une des revendications 1 à 5,
dans lequel le micro-canal (10) est équipé d'un recouvrement (40) qui ferme de façon définitive ou amovible le micro-canal (10).

7. Procédé d'obtention d'un modèle vasculaire conforme à l'une des revendications 1 à 6,
comprenant les étapes consistant à :
- fabriquer au moins un micro-canal (10) ayant une section partiellement circulaire par déformation d'une feuille de façon à obtenir une structure creuse (11) ayant une épaisseur de paroi de 0,1 µm à 1000 µm, cette feuille comportant déjà au moins un pores (12) avant la déformation et/ou après la déformation au moins un pore (12) étant introduit ou réalisé dans la feuille, et dans la structure creuse étant réalisé un ensemble de pores (12) dans les parois du canal, dont le diamètre est situé dans la plage de 1 nm à 100 µm,
- préparer au moins une chambre (20), et
- insérer le micro-canal sur la totalité de sa longueur et de sa largeur ou sur des parties (10) de celles-ci dans la chambre (20).

8. Procédé conforme à la revendication 7,
comprenant l'étape supplémentaire consistant à mettre en place un recouvrement (40) sur le micro-canal (10) pour permettre de le fermer en permanence ou de façon amovible.

9. Utilisation d'un modèle vasculaire conforme à l'une des revendications 1 à 6,
pour permettre la culture des structures de tissu tubulaires sur la face interne à courbure concave du micro-canal (10) et/ou sur la face externe à courbure convexe du micro-canal (10) et/ou dans la chambre (20).

10. Utilisation conforme à la revendication 9,
pour permettre l'imitation, la reproduction ou la simulation de vaisseaux sanguins et lymphatiques de la barrière sang-cerveau, d'épithélium de poumons, d'estomacs ou d'intestins et/ou de structures glandulaires.
